# EUROPEAN PATENT SPECIFICATION

(11) **EP 1 140 022 B1**
(45) Date of publication and mention of the grant of the patent: **13.02.2008**
(21) Application number: 99963103.9
(22) Date of filing: 17.12.1999
(51) Int. Cl.: A61K 9/127, A61K 31/704, A61K 31/137, A61K 47/48, A61P 35/00

(54) **METHOD OF ADMINISTERING A COMPOUND TO MULTI-DRUG RESISTANT CELLS**
VERFAHREN ZUR VERABREICHUNG VON EINER VERBINDUNG AN MEHRFACHRESISTENTE ZELLEN
PROCEDE D'ADMINISTRATION D'UN COMPOSE A DES CELLULES RESISTANT A PLUSIEURS MEDICAMENTS

(30) Priority: 18.12.1998 US 113004 P
(43) Date of publication of application: 10.10.2001
(73) Proprietor: HADASIT MEDICAL RESEARCH SERVICES & DEVELOPMENT COMPANY, LTD., 91120 Jerusalem (IL); ALZA CORPORATION, Mountain View, CA 94039-7210 (US)
(72) Inventor: GABIZON, Alberto, A., 96920 Jerusalem (IL); ZALIPSKY, Samuel, Redwood City, CA 94061 (US); GOREN-RUBEL, Dorit, 93389 Jerusalem (IL); HOROWITZ, Aviva, T., 92548 Jerusalem (IL)
(74) Representative: Hallybone, Huw George
(86) International application number: PCT/US1999/030230
(87) International publication number: WO 2000/035422

(56) References cited:
- WO-A-98/13072
- WO-A-98/16202
- US-A- 5 108 921
- US-A- 5 661 025
- PFLEIDERER W AND ROKOS H (EDS): "Chemistry and Biology of Pteridines and Folates, 11th Symposium 1997, Berchtesgaden" 1997 , BLACKWELL WISSENSCHAFTS-VERLAG GMBH, BERLIN , ISBN 3-8263-3185-0 XP000901874 page 353 -page 356 & HOROWITZ AT, ET AL.: "Folate-targeted liposomes with entrapped doxorubicin; binding and cytotoxicity studies in M109 murine lung carcinoma" the whole document
- GOREN E, ET AL.: "In-vitro and in-vivo studies of folate-targeted liposomes" PROCEEDINGS OF THE INTERNATIONAL SYMPOSIUM ON CONTROLLED RELEASE OF BIOACTIVE MATERIALS, vol. 24, 1997, pages 865-866, XP002140283 ISSN 1022-0178
- LEE RJ AND LOW PS: "Folate-mediated tumor cell targeting of liposome-entrapped doxorubicin in vitro" BIOCHIMICA ET BIOPHYSICA ACTA, vol. 1233, 1995, pages 134-144, XP000901581 ISSN 0005-2736
- MISLICK KA, ET AL.: "Transfection of folate-polylysine DNA complexes: evidence for lysosomal delivery" BIOCONJUGATE CHEMISTRY, vol. 6, no. 5, 1995, pages 512-515, XP000542461 ISSN 1043-1802
- R.J. LEE AND L. HUANG: "Folate-targeted, anionic liposome-entrapped polylysine-condensed DNA for tumor cell-specific gene transfer" J. BIOLOGICAL CHEM., vol. 271, no. 14, 5 April 1996 (1996-04-05), pages 8481-8487,
- C. P. LEAMON AND P. S. LOW: "Delivery of macromolecules into living cells: a method that exploits folate receptors endocytosis" PROC. NAT. ACAD. SCI. USA, vol. 88, July 1991 (1991-07), pages 5572-5576,
- S. WANG, R.J. LEE, ET AL.: "Delivery of antisense oligodeoxyribonucleotides against the human epidermal growth factor receptor into cultured KB cells with liposomes conjugated to folate via PEG" PROC. NAT. ACAD. SCI. USA, vol. 92, 1995, pages 3318-3322,
- LEE R.J. AND HUANG L.: "Folate-targeted, anionic liposome-entrapped polylysine-condensed DNA for tumor cell-specific gene transfer" J. BIOLOGICAL CHEM., vol. 271, no. 14, 5 April 1996 (1996-04-05), pages 8481-8487,

## Description

### Field of the Invention

The present invention relates to a method for administration of a therapeutic compound to multi-drug resistant cancer cells.

### Background of the Invention

After heart disease, cancer is the leading cause of death in the U.S. With the present methods of treatment, about one-third of patients are cured with local measures, surgery or radiation therapy, which are generally effective when the tumor has not metastasized by the time of treatment. In the remaining cases, early micrometastasis is a characteristic feature of the neoplasm, indicating that a systemic approach, such as chemotherapy, is required, often along with surgery or radiation.

One problem with cancer chemotherapy is drug resistance. Some tumor types, *e.g.*, non-small cell lung cancer and colon cancer, exhibit primary resistance, *i.e.*, absence of response on the first exposure to currently available, conventional chemotherapeutic agents. Other tumor types exhibit acquired resistance, which develops in a number of drug-sensitive tumor types. Drug resistant cancer cells demonstrate two types of acquired drug resistance; cells exhibiting single agent resistance or resistance to single class of anti-cancer drugs with the same mechanism of action. The second type involves cells broadly resistant to several or many chemically diverse anti-cancer drugs with different mechanisms of action. This second type of acquired resistance is known as multi-drug resistance.

Multi-drug resistance is also found in some tumor cell types, such as renal and colon tumors, exhibiting primary resistance. That is, in contrast to an acquired multi-drug resistance, certain tumor types are non-responsive to initial treatment with many chemotherapeutic agents.

Multidrug-resistance is often associated with increased expression of a normal gene, the *MDR1* gene, for a cell surface glycoprotein, P-glycoprotein, involved in drug efflux. P-glycoprotein expression correlates with a decrease in intracellular drug accumulation; that is, the P-glycoprotein acts as an energy-dependent pump or transport molecule that removes drugs from the cell, preventing the drug from accumulating in the cell.

P-glycoprotein is normally primarily expressed at epithelial and endothelial surfaces and seems to play a role in absorption and/or secretion. It is an active transporter that pumps hydrophobic drugs out of cells, reducing their cytoplasmic concentration and therefore toxicity. In normal cells, P-glycoprotein functions to eliminate toxic metabolites or xenobronc compounds from the body (Endicott, J. and Ling, V., Annu. Rev. Biochem., 58:137-171, (1989)).

Cancers which express P-glycoprotein include cancers derived from tissues which normally express the *MDR1* gene, namely cancers of the liver, colon, kidney, pancreas and adrenal. Expression of the gene is also seen during the course of thermotherapy with multidrug-resistant drugs in leukemias, lymphomas, breast and ovarian cancer, and many other cancers. These cancers initially respond to chemotherapy, but when the cancer relapses, the cancer cells frequently express more P-glycoprotein. There are cancers derived from tissues which do not normally express P-glycoprotein but in which P-glycoprotein expression increases during the development of the cancer. One example is chronic myelogenous leukemia, which when it goes into blast crisis, expresses more P-glycoprotein irrespective of the previous treatment history (Gorresman. M.M. Cancer Research, 53:747-754 (1993)).

The *MDR1*-encoded P-glycoprotein pump recognizes and transports many different substances, including most natural product anti-cancer drugs such as doxorubicin, daunorubicin, vinblastine, vincristine, actinomycin D. pacimaxel, and exoposide (Gorresman, M. et al. Current Opinion in Genetics and Development, 6:610-617 (1996)). More generally, the drugs often involved in multidrug-resistance are alkaloids as antibiotics of plant or fungal origin, and they include the vinca alkaloids, anthracyclines, epipodophyllotoxins and dactinomycin. Cross-resistance to alkylating agents such as melphalan, nitrogen mustard, and mitomycin C is occasionally observed (Endicott. J. and Ling, V., Annu. Rev. Biochem., 58:137-171.(1989)).

A. T. Horowitz et al. ("Chemistry and Biology of Pteridines and Folates, 11th Symposium 1997, Berchtesgadenn, eds W. Pfleiderer and H. Rokos, Blackwell Wissenschaftsverlag GmbH, Berlin, pages 353-356) relates to the cytotoxicity and binding of folic acid targeted liposomes with entrapped doxorubicin in cells susceptible and resistant to the drug. The authors report that liposomes with folic acid targeting ligands do not bind to membrane-associated folate receptors, but to an unidentified folate-receptor binder present in the extracellular matrix.

D. Goren et al. (Proceedings of the International Symposium on Controlled Release of Bioactive Materials, vol. 24, 1997, pages 865-866) relates to targeting drug-laden liposomes to the folate receptor of tumor cells by means of a folic acid targeting ligand. The authors report that a folate ligand coupled to liposomes improves binding of the liposomes to tumor cells, thereby enhancing cytotoxicity.

R.J. Lee et al (Biochemica et Biophysica Acta, vol 1233, 1995 pages 134-144) relates to targeting a drug, doxorubicin, to cancer cells by entrapping doxorubicin in folate-targeted liposome WO-98/16202 relates to delivery of a liposome-entrapped compound to the cytoplasm of a cell by fusion of the liposome lipid bilayer with the cell membrane. In one embodiment, a folate ligand is attached to the liposome to target the liposome to folate receptors on epithelial cells.

WO-98/13072 is concerned with antisense oligonucleotides which prevent expression of the *MDR1* gene in order to eliminate multiple drug resistance. In one embodiment, the antisense oligonucleotide is conjugated to a targeting molecule, such as folate, to achieve cellular delivery of the oligonucleotide via folate receptor endocytosis.

K.A. Mislick et al. (Bioconjugate Chemistry, vol. 6, no. 5, 1995, pages 512-515) is concerned with intracellular delivery of DNA via folate receptor endocytosis.

Clearly, multidrug-resistance in cancer cells limits successful chemotherapy and suggest a pour patient prognosis. One approach that has described to overcome multidrug resistance include coadministration of calcium channel blockers, such as verapamil, which inhibit the drug transport action of P-glycoprotein with the chemotherapeutic agent. This approach has not yet been proven in humans, and other strategies for overcoming multi-drug resistance are needed.

### Summary of the Invention

It is the object of the invention to provide a composition for administration of an anti-cancer therapeutic agent to multi-drug resistant cells according to claim 1.

These and other objects and features of the invention will be more fully appreciated when the following detailed description of the invention is read in conjunction with the accompanying drawings.

### Brief Description of the Drawings

Fig. 1 is a synthetic reaction scheme for the preparation of a folic acid-PEG-DSPE conjugate, showing the structure of the γ-carboxyl-linked conjugate;
Fig. 2 shows the results of a competitive binding study to determine binding of radiolabeled folic acid at a concentration of 0.1 µm to cellular folate receptor in murine lung carcinoma cell with a high density of folate receptor (M109R-HiFR) in the presence of the following competitors: free folate (open circles), free PEG₂₀₀₀ (closed squares) or a PEG-folate conjugate (closed triangles) at varying concentrations;
Figs. 3A-3F are schematic illustrations of the liposome compositions prepared in support of the present invention;
Figs. 4A-4B shows binding of various folic acid bearing liposomes and control liposomes (no folic acid ligand) to murine lung carcinoma cells with high (M 109-HiFR) and low (M109-LoFR) density of folate receptor (Fig. 4A) and to human epidermal carcinoma cells having a high density of folate receptor (KB-HiFR) and a low density of folate receptor (KB-LoFR) (Fig. 4B);
Figs. 5A-5D are confocal microscopic images of M109-HiFR cells incubated with rhodamine-labeled, folate targeted liposomes (Figs. 5A-5B) and with rhodamine-labeled, folate targeted liposomes having additional PEG chains (Figs. 5C-5D);
Figs. 6A-6B are confocal microscopic images of M109-HiFR cells incubated with rhodamine-folic acid-PEG₂₀₀₀ liposomes (HSPC/Chol/DSPE-PEG-Folate/DPPE-rhodamine (98.9:70:1.0:0.1)) for 30 minutes (Fig. 6A) and for 50 minutes (Fig. 6B);
Figs. 7A-7B are confocal microscopic images of M 109-HiFR cells incubated with rhodamine-folic acid-PEG₂₀₀₀ liposomes and with 2 mM free folate incubated for 4 hours (Fig. 7A) and for 19 hours (Fig. 7B);
Figs. 8A-8B are confocal microscopic images of M 109-HiFR cells (Fig. 8A) and M019-HiFR cells treated with phosphatidylinositol phospholipase C (Fig. 8B)) and incubated with rhodamine-folic acid-PEG₂₀₀₀ liposomes for 1 hour;
Figs. 9A-9F are images of M109R-HiFR cells (a subline of M109 cells selected for multi-drug resistance) incubated with free doxorubicin for 7 minutes (Fig. 9A) and for 30 minutes (Fig. 9B); with doxorubicin-loaded, folate-targeted liposomes for 20 minutes (Fig. 9C), 60 minutes (Fig. 9D) and for 90 minutes (Fig. 9E); and with non-folate-targeted liposomes coated with mPEG-DSPE (known commercially as DOXIL) for 4 hours (Fig. 9F);
Figs. 10A-10D are images of M109R-HiFR cells incubated for 1 hour with free doxorubicin (Fig. 10A) or with folate-targeted liposomes containing doxorubicin (Fig. 10B) and for 24 hours in drug free medium after 1 hour of incubation with free doxorubicin (Fig. 10C) or with folate-targeted liposomes containing doxorubicin (Fig. 10D);
Figs. 11A-11B are results from the flow cytometry assay for M109R-HiFR cells exposed to free doxorubicin (Fig. 11A) and to folate-targeted liposomes carrying doxorubicin (Fig. 11B) in the presence or absence of verapamil, a P-glycoprotein blocker;
Fig. 12 is a plot showing the kinetic of doxorubicin uptake by M109R-HiFR cells exposed to doxorubicin entrapped in folate-targeted liposomes at drug-to-lipid ratios of 137.6 µg/µmol (closed circles) and 11.3 µg/µmol (open circles);
Fig. 13 is a bar graph showing the accumulation of doxorubicin in M109R-HiFR cells after exposure to free doxorubicin and doxorubicin entrapped in folate-targeted liposomes for 1 hour and for 4 hours, where the accumulation was determined for the cell nuclei and cytosol;
Figs. 14A-14B show cytotoxicity results for M109-HiFR (Fig. 14A) and M109R-HiFR (Fig. 14B) cells when exposed to doxorubicin in free form (closed circles) or in folate-targeted liposome-entrapped form (closed triangles) or in non-folate-targeted liposome-entrapped form (closed squares);
Figs. 15A shows the time course of mean footpad thickness, in mm, after injection of tumor cells into the footpad of mice, where the tumor cells were treated *in vitro* prior to injection with free doxorubicin (closed circles), liposome-entrapped doxorubicin (open squares) or with folate-targeted liposomes (closed triangles). The control mice received untreated tumor cells (open circles);
Fig. 15B shows the mean footpad tumor weight, in grams, on day 34 of the test animals of Fig. 15A, where the footpad tumor weight is taken as the weight of the footpad on day 34 minus the average footpad weight of a healthy mouse; and
Fig. 16 is a plot showing the number of palpable- tumors after subcutaneous injection of untreated (control, open circles) or *in vitro* treated tumor cells with free doxorubicin (closed circles), doxorubicin entrapped in non-targeted liposomes (open squares) and doxorubicin entrapped in folate-targeted liposomes (closed triangles).

### Detail Description of the Invention

The invention in one aspect, is directed to a composition for administration of a therapeutic agent to a multi-drug resistant cell. In practice, the composition provides for administration of a therapeutic agent to a person suffering from cancer, and in particular from a cancer which expresses P-glycoprotein on the cancer cell surfaces. As noted above, certain cancers, such as renal cancer and colon cancer exhibit primary resistance, as opposed to secondary or refractory resistances, to many chemotherapeutic agents. The composition and method of the invention provides for treatment of these cancers, as well as those cancers that are refractory, *e.g.* as in those cancer that develop multi-drug resistance where the cancer is initially responsive to a therapeutic agent or group of agents but progresses to a state that is no longer responsive to or successfully treatable by the agent(s).

In one aspect, the invention includes a composition composed of a carrier, a folate targeting ligand, and the drug tobe administered. The folate ligand is covalently attached to the carrier, and the drug is associated with the carrier. By associated, it is meant, that the drug is covalently or electrostatically attached, or is entrapped or encapsulated with the carrier. As will be described below, the composition is effective to achieve accumulation of the drug in multi-drug resistant cells, *i.e*., cells expressing the P-glyoprotein which acts as an efflux pomp to prevent accumulation of drug in the cell, in an amount sufficient to be cytotoxic to the cell. By "cytotoxic" it is meant that the amount of drug accumulated in the cells is sufficient to prevent normal cell functioning and, preferably, to cause cell death. Attached to the carrier, either at an end or along carrier

itself or attached to the surface of a microsphere prepared from the carrier, is a folate ligand, which will be described below. The drug to be administered is also attached to the carrier or is in some way associated with the carrier so that it moves with the carrier and the targeting folate ligand. As will be described below, the folate effectively targets the conjugate to a multi-drug resistant cell for delivery and accumulation of the drug in the cell.

The carrier is a liposome. In studies performed in support of the invention, folate-targeted liposomes were prepared and the general concept was demonstrated using the carrier liposomes. It will be appreciated, however, that the useful teaching from the liposome studies is applicable to a number of carriers, as will be apparent from the studies described below.

### I. Preparation of Folate-Receptor Targeted Liposomes

### A. Synthesis and Characterization of mPEG-folic acid and folic acid-PEG-DSPE

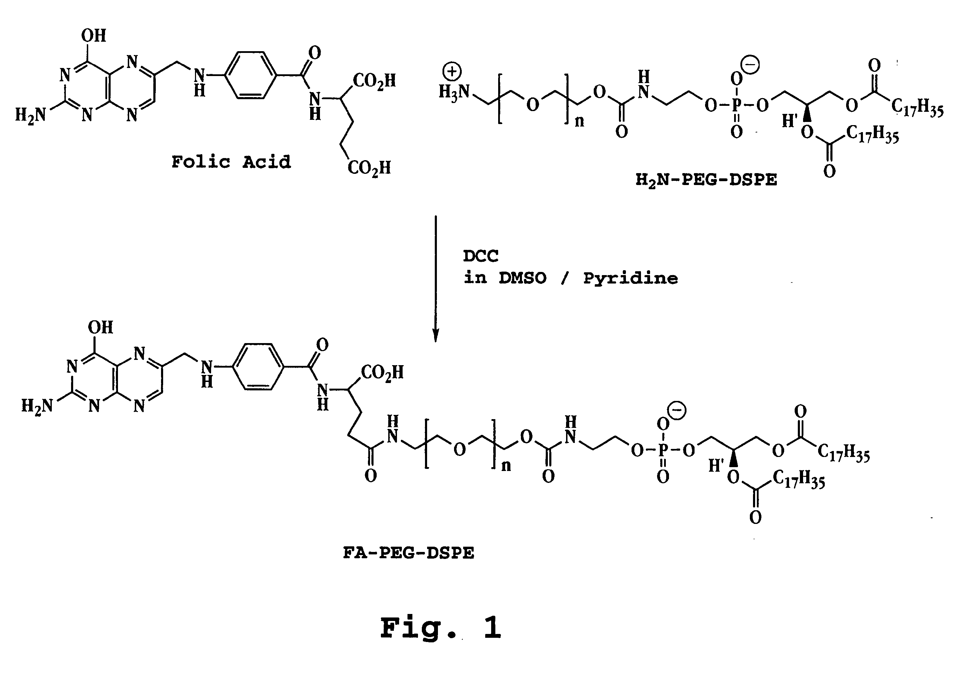

Conjugates composed of folate, polyethylene glycol (PEG) and distearoly phosphatidyl ethanolamine (DSPE) (folic acid-PEG-DSPE) were prepared as shown in Fig. 1, via a dicyclohexylcarbodiimide - mediated coupling of folic acid to H₂N-PEG-DSPE. As described in Example 1A, the starting amino-PEG lipids were synthesized from PEG having molecular weights 2000 Daltons and 3350 Daltons according to previously described methods (Zalipsky, S. et al., FEBS Lett. 353:71-74 (1994)). Conjugates of folic acid and methoxy-PEG, e.g., conjugates without the phospholipid moiety, were prepared using the same coupling method. The structures of the purified conjugates were corroborated by ¹H-NMR, MALDI-TOFMS and UV spectroscopy, as set forth in Examples 1B-1C. Carbodiimide-activated folic acid can couple with H₂N-PEG-DSPE via either α or γ-carboxyl groups of its glutamate residue. The γ-conjugate binds to the folate receptor, whereas the α-conjugate does not, therefore the relative amounts of each conjugate were determined by a method using carboxypeptidase G (CPG). As described in Example 1E, a method using carboxypeptidase G (CPG) was used, since it is known that α-conjugates are inert to the enzyme while the γ-conjugates are subject to pteroate-glutamate cleavage at an appreciable rate (Wang, S. et al., Bioconjugate Chem., 7:56-62 (1996); Fan, J., et al., Biochemistry, 30:4573-4580 (1991); levy C. and Goldman P., Biol Chem., 242:2933-2938 (1967)). The enzymatic cleavage was followed by disappearance of the conjugate peak by HPLC. This reaction proceeded up to 80% conversion despite prolonged incubation times and multiple additions of the enzyme, indicating that folic acid-PEG-DSPf- contained 80% γ-carboxy linked and the remaining 20% were α-linked conjugates. The same approach was used to determine their 90% of mPEG-folic acid was linked through the γ-carboxyl group.

The conjugates were characterized by binding studies, as will now be described. As set forth in Example 2, three of the cell lines used, the murine lung carcinoma (M109), a multidrug-resistant subline of M109 (M109R) and human nasopharyngeal epidermal carcinoma (KB) were induced to upregulate their folic acid receptors by consecutive passages in folic acid-depleted medium (3 nM folic acid). This resulted in three cell sublines referred in as "high folate receptor" (HiFR) with 20-80 fold increase in folic acid-binding capacity over the parental cell lines, which are referred to herein as low folate receptor" (LoFR).

These cell lines and their capacity to bind folic acid were determined as set forth in Example 2B, by incubating the cells for 30 minutes with folic acid at 37°C. Two of the cell lines, the M109-LoFR and KB-LoFR cells, were also assayed for folic acid binding after 24 hours of incubation in folic acid-depleted medium. The folate binding to normal human fibroblasts in early passage and to a human melanoma line A375 was also determined, to obtain a spectrum of different cell lines with a broad range of receptor expression levels. The results are summarized in Table 1.

**Table 1**

| CELL LINE | CELL LINE ABBREVIATION | FOLIC ACID BINDING^{a} FENTOMOLES/10⁴ CELLS |
|---|---|---|
| Murine Lung Carcinoma Low Folate Receptor | M109-LoFR | 20±6 |
| Murine Lung Carcinoma Low Folate Receptor | M109-LoFR^{b} | 25±13 |
| Multidrug-resistant murine lung carcinoma Low Folate Receptor | M109R-LoFR | 27±10 |
| Murine Lung Carcinoma High Folate Receptor | M109R-HiFR | 678±755 |
| Multuring-resistant High Folate Receptor | M109R-HiFR | 2179±430 |
| Nasopharyngeal epidermal carcinoma Low Folate Receptor | KB-LoFR^{b} | 6725±323 |
| Nasopharyngeal epidermal carcinoma High Folare Receptor | KB-HiFR | 9730±79 |
| Human Melanoma line A375 | A375 | 168±33 |
| Normal Human Fibroblasts | NHF | 45±3 |

| | | |
|---|---|---|
| ^{a} Incubation time, 30 min, at 37°C ^{b} After culture in folic acid-depleted medium for 24 h. | | |

When the line with the lowest (M109-LoFR) and highest (KB-HiFR) amount of receptors are compared, differences in folic acid binding capacity up to 485-fold were observed. Also as seen in Table 1, for the cell lines incubated for 24 hours in folic acid-depleted medium, M109-LoFR did not upregulate the amount of folic acid binding. In contrast, KB-LoFR showed a 15-fold increase in folic acid binding indicating rapid upregulation of receptor expression.

In further studies to characterize the binding of folic acid with the folate receptor-overexpressing M 109-HiFR cell line, the following observations were made:
i) binding is directly proportional to cell number in the range of 10³ to 1.5x10⁶ cells per plate;
ii) monolayer cultures of M109R-HiFR cells pretreated with phosphatidylinositol-phospholipase C (PI-PLC) (see Example 2B) lose 99 % of their folic acid receptors, as shown by binding assay with folic acid, indicating that the overexpressed folate receptor is bound to the cell membrane by a glycophospholipid anchor. In contrast, trypsin treatment does not damage the folate receptor, as indicated by (a) radiolabeled folic acid binds to a similar extent to plated cells and to suspension cells after trypsinization, and (b) cell-bound radiolabeled folic acid is almost fully recovered (91 ± 11 %) after trypsinization; and
iii) Only 2 ± 1 % of folic acid remains bound to M109R-HiFR cell monolayers following acid wash (pH 3), indicating that the binding of folic acid to the overexpressed receptors is pH-sensitive. To prevent internalization, the folic acid binding assay took place at 1°C for 30 min. When M109R-HiFR cells are incubated for 4 hours at 37°C with radiolabeled folic acid, and then submitted to acid wash, 30 to 40% of the ligand is retained by cells. This is most likely the fraction of folic acid ligand that is internalized by cells, thus avoiding the pH-induced dissociation from the receptor.

As described in Example 2B, a competition binding study was performed with folic acid, mPEG-folic acid, and free PEG. In this study, M109R-HiFR cells were exposed to a constant amount of radiolabeled folic acid at a concentration of 0.1 µM. "Cold" folic acid, mPEG-folic acid, and free PEG were added to the M109R-HiFR cells at concentrations varying from 0.1 to 100 µM. The PEG₂₀₀₀ and the mPEG₂₀₀₀-folic acid conjugate were freely water-soluble, not lipid linked, compounds. The latter derivative can be viewed as a monovalent version of folic acid-PEG₂₀₀₀-liposome as will be discussed below.

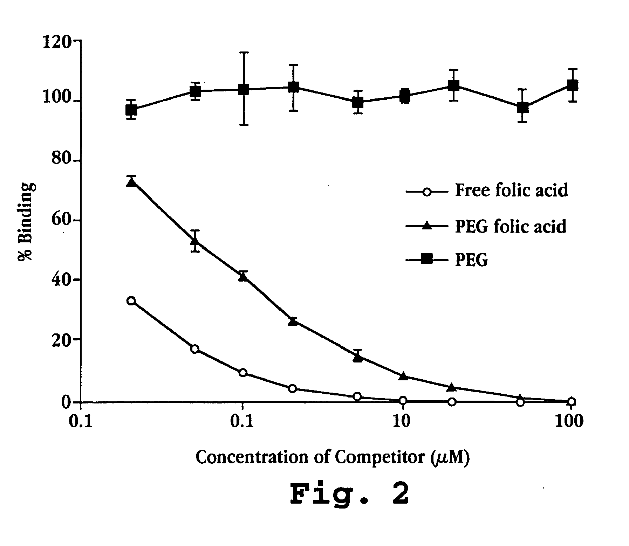

The results of the competitive binding assay are shown in Fig. 2 as percentage of binding against concentration in µm of each of the competitors, free folic acid (open circles). PEG-folic acid (closed triangles) and PEG (closed squares). As seen, mPEG-folic acid was less effective than free folic acid in competing with radiolabeled folic acid for binding to the folate receptors, suggesting that the attachment of PEG to the vitamin molecule diminishes by 5 to 10-fold the ability of folic acid to bind to the receptor. Free PEG exhibited no cell binding, showing no competition with folic acid binding.

### B. Liposome Preparation

Six liposome formulations were prepared according to the procedures set forth in Example 3. The six liposomal compositions are summarized in Table 2.

**Table 2**

| Components | Molar Ratio of Components | Abbreviation |
|---|---|---|
| HSPC:Chol:folic acid-PEG₂₀₀₀-DSPE | 99.5 : 70 : 0.5 | folic acid-PEG₂₀₀₀ |
| HSPC:Chol:folic acid-PEG₃₃₅₀-DSPE | 99.5 : 70 : 0.5 | folic acid-PEG₃₃₅₀ |
| HSPC:Chol:mPEG₂₀₀₀-DSPE | 99.5 : 70 : 0.5 | Low mPEG₂₀₀₀ |
| HSPC:Chol:folic acid-PEG₂₀₀₀-DSPE:mPEG-DSPE | 92.5 : 70 : 0.5 : 7 | folic acid-PEG₂₀₀₀/mPEG |
| HSPC:Chol:folic and-PEG₃₃₅₀-DSPE:mPEG-DSPE | 92.5 : 70 : 0.5 : 7 | folic acid-PEG₃₃₅₀/mPEG |
| HSPC:Chol:mPEG₂₀₀₀-DSPE | 92.5 : 70 . 7.5 | High mPEG₂₀₀₀ |

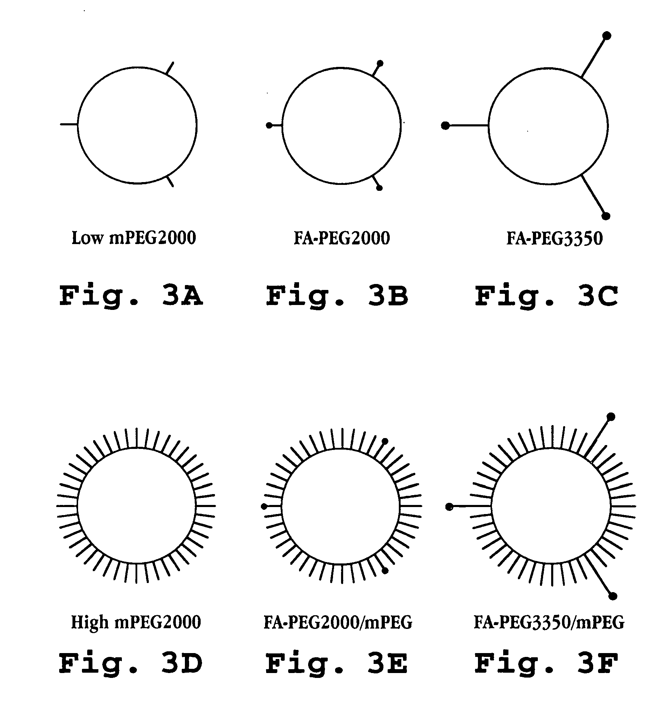

As can be seen in Table 2, all of the formulations contained HSPC, Chol, and DSPE, thus, they are referred to herein according to folic acid-PEG/mPEG content, as noted in the right-hand column of the table. Figs. 3A-3F schematically illustrate the liposomal formulations. Four folate-targeted liposome formulations were prepared (Figs. 3B. 3C, 3E, 3F); two formulations included a folic-acid-PEG conjugate prepared with PEG molecular weight of other 2000 Daltons (Fig. 3B; folic-acid-PEG₂₀₀₀ for 3350 Daltons (Fig. 3C; folic-acid-PEG₃₃₅₀ and two formulations that included the foloc-acid-PEG conjugate in addition to an mPEG-DSPE conjugate prepared with mPEG of molecular weight 2000 Daltons (Fig. 3E, folic acid-PEG₂₀₀₀/mPEG and Fig. 3F, folic-acid-PEG₃₃₅₀/mPEG). In all four formulations the folic acid-PEG- conjugate derived from PEG of molecular weight 2000 or 3350 Daltons was included at a molar fraction of 0.5% of total liposomal phospholipid. The two control formulations contained no folate (Figs. 3A and 3D) and differed in the mole fraction of mPEG₂₀₀₀₋DPSE included (0.5% and 7.5%, respectively).

In the studies in support of the invention, discussed below, it was found that the control liposome formulation, that is the non-targeted high-mPEG and low-mPEG formulations, behaved similarly *in vitro,* therefore, data are presented only for the high-mPEG liposomes.

### C. In vitro Binding Studies

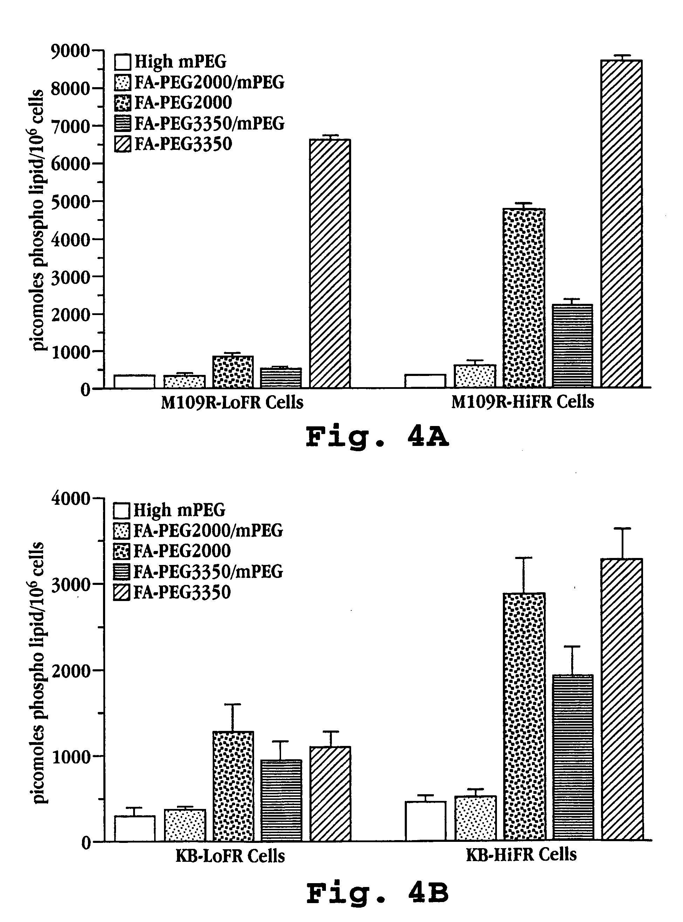

The binding of radiolabeled folic acid-targeted liposomes and the control, non-targeted liposomes was assayed on monolayer cultures or the multi-drug resistant cell lines with both high and low folate receptors level. M109R-HiFR and M109R-LoFR cells, at 37 °C for 24 hours, as described in Example 2B. The results are shown in Fig. 4A where the amount of liposomes bound to trypsin-released cell, expressed as picomoles phospholipid per million cells, is shown for each of the liposome formulations. As seen in the figure, binding of the non-targeted, conventional mPEG liposomes to the multidrug-resistant cells was very low for both high and low folic acid receptor levels. The targeted formulation with the highest binding efficiency was the liposomes having the folic acid-PEG₃₃₅₀ conjugate, which achieved a 26-fold increase in binding over the non-targeted liposomes. Accordingly, in one embodiment of the invention, the composition includes a liposomal carrier having PEG polymer chains with a molecular weight of at least about 3,500 Daltons, with a preferred range between 2,500-10,000 Daltons and a more preferred range between 3,500-10,000 Daltons. In the embodiment, the composition is effective to achieve at least a 10-fold, more preferably a 20-fold, and most preferably a 25-fold increase in binding compared to a composition having PEG chains with a molecular weight of less than about 2,500 Daltons, and specifically a molecular weight of 2,000 Daltons.

With continuing reference to Fig. 4A, addition of mPEG to the formulation or shortening of the PEG rather from 3350 to 2000 reduced the binding substantially. Binding was greater for M109R-HiFR cells than for M109R-LoFR cells for all of the liposome formulations. Interestingly, the highest affinity liposome formulation, e.g., liposomes having the folic: acid-PEG₃₃₅₀, showed the lowest relative increase of binding (-30%) when the LoFR and HiFR cells were compared.

Fig. 4B is a plot similar to Fig. 4A, except that the study was performed with the KB cell line. As seen in the figures, the addition of mPEG to the liposomes reduced the efficiency of binding. In the absence of mPEG, a longer PEG spacer (3350 Daltons) increased only slightly the efficiency of binding to KB cells. The differences between KB-HiFR and KB-LoFR were for some of the liposome compositions smaller than as seen for the M109R cells. This may be related to the rapid up-regulation of folate receptors in KB cells during the 24 hour incubation period, as discussed above with reference to Table 1.

To study if the cell-associated liposomes were cell-surface bound or internalized into cells, an acid wash with saline, pH 3, was performed at the end of the binding assay. The results, which are shown in the last column of Table 3, indicate that about 22% of folic acid-PEG₃₃₅₀ and 32 % of folic acid-PEG₃₃₅₀/mPEG liposomes were removed by the acid wash, which indicates that more than 2/3 of the cell-associated liposomes have been internalized by M109R-HiFR cells. Also shown in Table 3 are competition studies with 1 mM folic acid ("700-fold excess over the concentration of liposome-bound folic acid) added to the liposome/cell mixture, either at the beginning or at the end of the incubation of the liposomes with the cells. As seen in the table, folic acid added at the beginning of the incubation period of the targeted FA-PEG₃₃₅₀ liposomes with the M109R-HiFR cells only partially inhibits (46%) liposome binding. Addition of the folic acid at the end of the 24 hour liposome/cell incubation, results in no significant displacement of folic acid-bearing liposomes from the cells, despite the fact that, according to acid wash experiments, approximately 20-30% of the liposomes were still bound to the cell surface. These studies demonstrate the greater binding avidity of folic acid-PEG liposomes over free folic acid for the cellular folate receptor, which is in contrast to 5-10 fold less efficient binding of the monovalent analog, mPEG-folic acid (Table 1). The stronger binding of folic acid-targeted liposomes is related to the multivalent presentation of folic acid residues on the liposomal platform.

**Table 3**

| **ADDITIONAL TREATMENT** | **1MM FA AT TIME 0(A)** | | **1MM FA AT TIME 24H (B)** | | **ACID WASH AT 24H (C)** | |
|---|---|---|---|---|---|---|
| Liposome Composition | FA-PEG | FA-PEG / mPEG | FA-PEG | FA-PEG / mPEG | FA-PEG | FA-PEG / mPEG |
| % inhibition | 46±8 | 86±6 | 2±3 | 8±4 | 22±2 | 32±5 |

| | | | | | | |
|---|---|---|---|---|---|---|
| (a) M109R-HiFR cells were incubated for 24 h with 300 nmol/mL FA-PEG3350- or FA-PEG3350/mPEG-coated liposomes in the presence or absence of free FA (b) or FA added for 2 h after 24 h incubation with liposomes (c) or cells washed with acid saline (pH 3) | | | | | | |

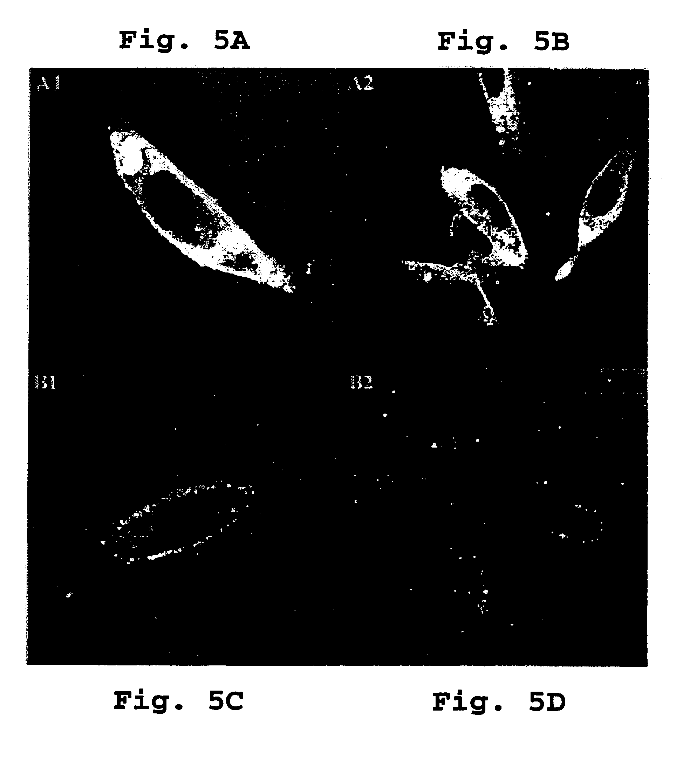

As noted above, these studies suggested that liposome formulations which include mPEG-DSPE in addition to the PEG chains having the attached folate ligand, e.g., the folic acid-PEG₂₀₀₀/mPEG and folic acid-PEG₃₃₅₀/mPEG formulations, interfere with binding to the target cells. This suggestion was further examined by exposing cells to rhodamine-labeled liposomes, prepared as described in Example 4,-and viewing the cells by confocal microscopy. M109-HiFR cells were incubated to rhodamine-folic acid-PEG₂₀₀₀ liposomes (HSPC/Chol/DSPE-PEG-Folate/DPPE-rhodamine (98.9:70:1.0:0.1)) or with rhodamine-folic acid-PEG₂₀₀₀/mPEG₂₀₀₀ liposomes (HSPC/Chol/mPEG-DSPE/DSPE-PEG-Folate/DPPE-rhodamine (91.9:70:7:1.0:0.1)) for 7 hours and examined by confocal microscopy. The results are shown in Figs. 5A-5B and Figs. 5C-5D for the rhodamine-folic acid-PEG₂₀₀₀ liposomes and the rhodamine-folic acid-PEG₂₀₀₀/mPEG₂₀₀₀, respectively. As seen in Figs. 5A-5B, the cytoplasm of M109-HiFR cells exposed to folate-targeted liposomes with no additional PEG chains are loaded with rhodamine-labeled liposomes. In contrast, as seen in Figs. 5C-5D, liposomes with the additional mPEG have poor binding to the cells, as evidenced by the faint signal of rhodamine associated with the cell surface and the failure of the liposomes to internalize into the cells. These results confirm that the folate ligand is able to mediate binding and internalization of folate-targeted liposomes to cells with overexpressed folate receptors and that the additional PEG chains appear to interfere with these processes.

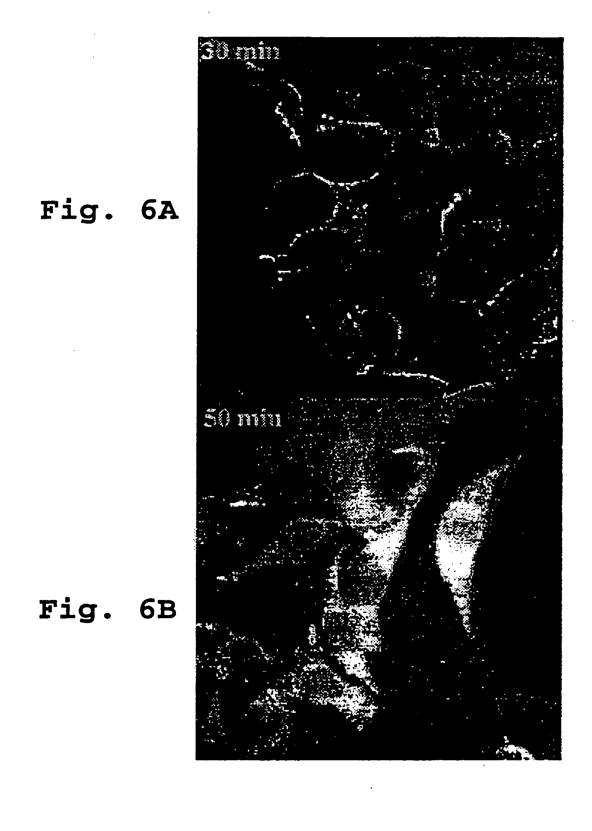

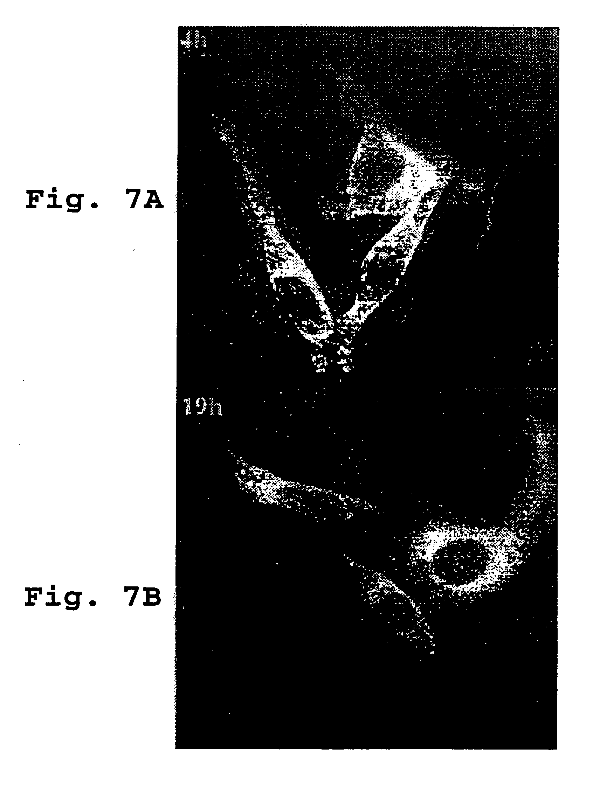

In another study performed in support of the invention, M 109-HiFR cells were incubated with rhodamine-folic acid-PEG₂₀₀₀ liposomes (HSPC/Chol/DSPE-PEG-Folate/DPPE-rhodamine (98.9:70:1.0:0.1)) in folate-free medium. After 30 minutes and after 50 minutes of incubation, the cells were visualized with confocal microscopy and the images are shown in Figs. 6A-6B. As seen in Fig. 6A, after 30 minutes of incubation, the folate-targeted liposomes are associated with the M109-HiFR cells, and after 50 minutes, as seen in Fig. 6B, some liposomes are internalized and accumulate in the cell cytosol. In the same study, some cells were also exposed to free folic acid at a concentration of 2 mM, which was equivalent to about a 1000 times the concentration of liposomal-bound folate. This competitive binding study was conducted to verify interaction between the folate ligand and the folate receptor. After 30 minutes and 50 minutes of incubation, the cells were visualized and no liposome binding was observed (Fig. 6), suggesting that the free folate was able to competitively block binding of the folate-targeted liposomes to the cellular folate receptor. After longer exposure times of 4 hours and 19 hours folate-targeted liposome binding to the M109-HiFR cells was no longer blocked by the free, soluble folate and the cell cytoplasm was stained with rhodamine fluorescence, as seen in Figs. 7A-7B.

Without being bound to any particular theory, the results of the competitive binding study may be due to the fact that affinity of binding is higher for liposomal folate than for free folate, in view of the multivalency of the liposomes. However, equilibrium for the folate liposomes is teached after a longer period of time, particularly in view of the large excess (1000 fold) of free folate and the more rapid mobility of small molecules as compared to nanoparticles (liposomes).

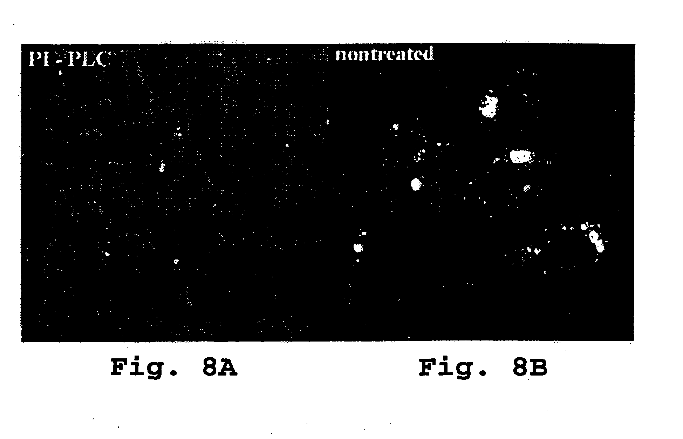

Further evidence of the involvement of the folate receptor with the folate-targeted liposomes with M109-HiFR cells is provided by the studies conducted with cells pretreated with phosphatidylinositol-phospholipase C (PI-PLC) (Example 2B) to destroy the folate receptor. Exposure of PI-PLC pretreated M109-HiFR cells to rhodamine-labeled, folate-targeted liposomes for 1 hour at 4°C resulted in no detectable binding as seen in the image of Fig. 8A. The same liposomes, however, were bound by non-Pi-PLC-treated cells, as seen in the image of Fig. 8B. At 4°C binding to the receptor occurs but internalization and recycling of the receptor to the cell surface do not occur (Kamen B.A. and Capdevila A., Proc. Natl. Acad. Sci. USA., 83:5983-5987 (1986)). The inability of folate-targeted liposomes to associate with the enzyme-treated M109 HiFR cells provides evidence that the glycophospholipid-anchored folate receptor is involved in liposome-cell association.

### 1. In vitro Binding Studies Using Doxorubicin Loaded Liposomes

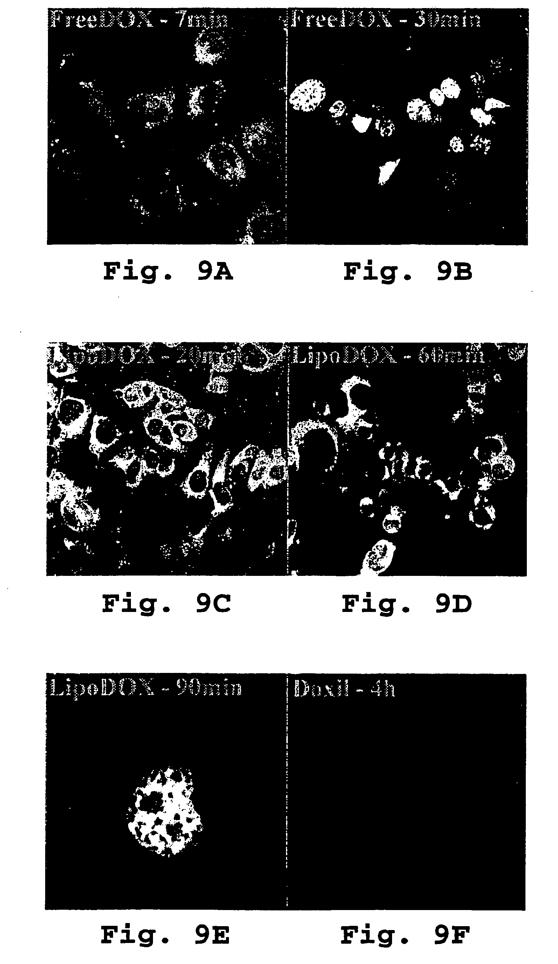

As described in Example 5, folate-targeted liposomes containing doxorubicin were prepared and incubated with M109R-HiFR cells. For comparison, M109R-HiFR cells were also incubated with free doxorubicin or with liposomes containing doxorubicin but with no folate targeting ligand. The movement of the doxorubicin molecule was tracked using fluorescence and the results are shown in Figs. 9A-9F.

Figs. 9A-9B are images for cells exposed to free doxorubicin for 7 minutes (Fig. 9A) and for 30 minutes (Fig. 9B). The influx of free doxorubicin through the cell membranes was very rapid as indicated by the bright cytoplasmic staining in Fig. 9A. As can be seen in Fig. 9B, the free drug was already located in the nucleus within 30 minutes.

The kinetics of cell interaction with doxorubicin-loaded, folate-targeted liposomes wax considerably different from that with free doxorubicin As seen in Figs. 9C-9E, liposome attachment to the cell membrane was observed within approximately 20 minutes (Fig. 9C). By 60 minutes, internalization has taken place and liposomal doxorubicin was detected in the cytosol and, in a few cells, the drug began to appear in the nucleus (Fig. 9D). After 90 minutes, doxorubicin delivered from folate-targeted liposomes has reached the nucleus in most of the cells while the cytoplasmic drug fluorescence has disappeared (Fig. 9E). In contrast to folate-targeted liposomes, a formulation of non-targeted liposomes coated with mPEG-DSPE (known commercially as DOXIL) showed no association with the M109R-HiFR cells, as seen in Fig. 9F, even after 4 hours of incubation. This study was repeated with fresh and fixed cells with essentially similar results.

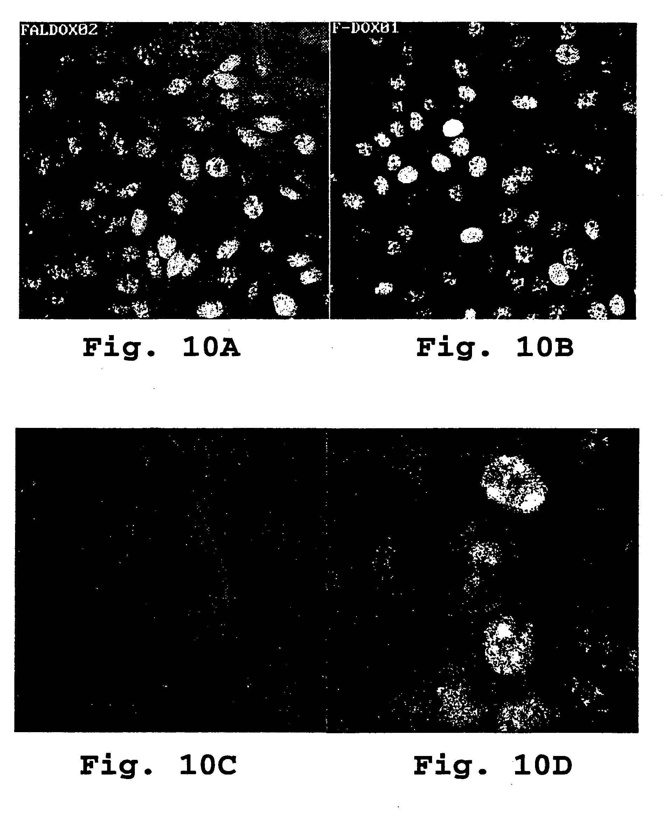

In another study, the ability of the M 109R-HiFR cells to retain doxorubicin after treatment for 1 hour with either free doxorubicin or with doxorubicin-loaded folate-targeted liposomes, followed by incubation in drug-free medium for 24 hours was examined. As seen in Figs. 10A-10D, the cell nuclei are stained by doxorubicin fluorescence after 1 hour of incubation with both the free drug (Fig. 10A) and with the liposome-entrapped drug (Fig. 10B). However, after 24 hours the fluorescence has almost completely waned in the cells treated with the free drug, as seen in Fig. 10C. The fluorescence in the cells treated with the liposomes is still clearly detectable, as seen in Fig. 10D. Thess results shows that liposomal drug is retained in the drug resistant cells better than free drug.

### 2. Further Evidence of Drug Accumulation in MDR Cells

As described in Example 6, studies were performed to show that intracellular delivery of doxorubicin via the folate receptor pathway in the form of folate-targeted doxorubicin-carrying liposomes overcomes the P-170 glycoprotein efflux pump. In one study, the activity of the P-170 glycoprotein pump in M109R-HiFR cells was examined by flow cytometry using a rhodamine efflux assay and was found to be sensitive to verapamil blockade (data not shown). Using this technique, the efflux of doxorubicin intracellularly delivered in free form and in folate-targeted liposome-entrapped form was examined. Monolayers of M109R-HiFR cells were exposed to doxorubicin in free form or in liposome-entrapped form for 1 hour in the presence of verapamil. The cells were rinsed and incubated again in 10 µmol verapamil for 2 hours. The cells were then analyzed for cellular doxorubicin content using fluorescence and by flow cytometry.

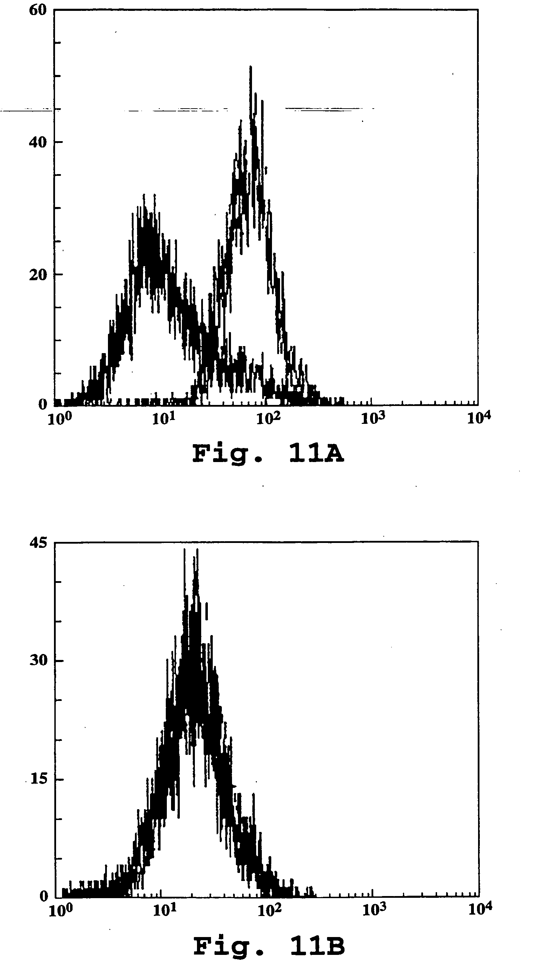

The results from flow cytometry are shown in Figs. 11A-11B for the cells treated with free doxorubicin (Fig. 11A) and with liposome-entrapped doxorubicin (Fig. 11B). As seen in Fig. 11A, the curve shift indicates a clear increase of cell fluorescence in M109R-HiFR cells after exposure to free doxorubicin in presence of verapamil. In contrast, the cellular level of fluorescence in M 109R-HiFR cells upon exposure to the folate-targeted liposomal drug appears unchanged in the presence or absence of verapamil (Fig. 11B). These observations were confirmed by quantitative fluorometry of doxorubicin from cell extracts as summarized in Table 4.

**Table 4**

| **Exposure Time (minutes)** | **Intracellular Doxorubicin Content** | | | |
|---|---|---|---|---|
| | **ng/1.5 x 10⁶ cells** | | | |
| | **Free Doxorubicin** | **Free Doxorubicin + verapamil** | **Targeted, liposomal Doxorubicin** | **Targeted, liposomal Doxorubicin + verapamil** |
| 30 | 24 | 89.8 | 155.7 | 151.5 |
| 60 | 42.5 | 166.5 | 198.5 | 192.2 |

As seen in the table, the presence of verapamil had no effect on the amount of drug accumulating in the drug-resistant cells when delivered via folate-targeted liposomes. In contrast, cell retention of doxorubicin administered in free form was -4.5 fold higher in the presence of verapamil. Drug retention is approximately 4-6 fold higher when administered from folate-targeted liposomes. These results indicate that free doxorubicin diffusing into the cells is pumped out by P-170 glycoprotein pump action, while doxorubicin entry via the folate receptor pathway avoids the P-glycoprotein efflux machinery.

In an additional study to confirm enhancement of drug delivery to cells via folate-targeted liposomes, drug-resistance (M109R) cells and drug-sensitive (M109) cells were exposed to 0.2 x 10⁻⁵ M and 0.5 x 10⁻⁶ M doxorubicin, respectively. The doxorubicin was administered to the cells in free form and entrapped in folate-targeted liposomes. The cell-associated drug was measured after 1 hour and 4 hours of exposure to doxorubicin. The results are shown in Table 5.

**Table 5**

| **Percent Doxorubicin in Cells After Adminstration and Amount of Doxorubicin in Cells** | | | | |
|---|---|---|---|---|
| **Exposure Time (hours)** | **Percent doxorubicin administered remaining in cells¹ and intracellular doxorubicin (ng/1.5 x 10⁶ cells)²** | | | |
| | **Drug-Resistant Cells** | | **Drug Sensitive Cells** | |
| | **Free Doxorubicin** | **Targeted, liposomal Doxorubicin** | **Free Doxorubicin** | **Targeted, liposomal Doxorubicin** |
| 1 | **5.5** (159) | **13.2** (385) | **8.6** (25) | **12.7** (37) |
| 4 | **4.2** (122) | **20.5** (595) | **12.4** (36.1) | **22** (64) |

| | | | | |
|---|---|---|---|---|
| ¹ values in bold are percent of doxorubicin administered remaining in cell ² values in parenthesis are ng of doxorubicin in the cells | | | | |

The data is Table 5 shows that drug-resistant cells exposed to free doxorubicin pump out the drug, even in the presence of excess drug in the extracellular medium bathing the drug. In contrast, drug resistant cells exposed to doxorubicin in the form of folate-targeted liposomes accumulate doxorubicin in the cells. As expected, the drug sensitive cells are able to accumulate doxorubicin in both free form and in liposome-entrapped form. These data show that while drug-resistant cells exposed to free doxorubicin effectively pump out the drug, drug delivery and accumulation in the cells effectively occurs when delivered via the folate pathway.

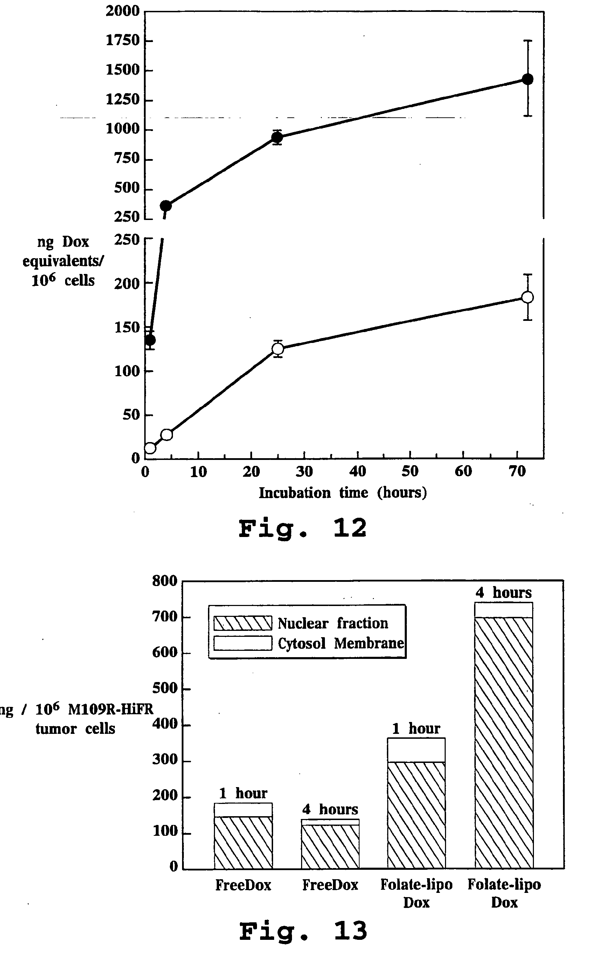

The dependence of drug uptake and accumulation in drug-resistant cells on the folate pathway is further supported by the study presented in Fig. 12. In this study, two formulations of folate-targeted liposomes having entrapped doxorubicin were prepared. One formulation had a drug-to-lipid ratio of 137.6 µg/µmol (closed circles in Fig. 12) and the other had a drug-to-lipid ratio of 11.3 µg/µmol (open circles). M109R-HiFR cells were incubated with the liposome formulations and the uptake of drug into the cell was monitored as a function of time. As seen in Fig. 12, the cellular accumulation of doxorubicin was consistently higher by a factor of -10 for the liposomes having the 10-fold higher drug-to- ' lipid ratio. The steady accumulation of targeted liposomal drug by tumor cells during the 24-hour incubation period, without evidence of efflux and without plateau level, is also apparent from the curves in Fig. 12.

In another study, the quantitative amount of doxorubicin accumulated in the cell nucleus and the cell cytosol was determined via cell fractionation. M109R-HiFR cells were incubated with free doxorubicin or with doxorubicin entrapped in folate-targeted liposomes for 1 hour and for 4 hours. Accumulation of doxorubicin in the cell nuclei and cytosol was measured fluorimetrically after separation of the cell nuclei from the cytosol, as set forth in Example 7. The results are shown in Fig. 13, where as seen, after 1 hour of incubation, most of the drug is found in the nuclear fraction with both free doxorubicin (DOX) and targeted-liposomal doxorubicin. After 4 hours of incubation the nuclear drug concentration obtained with targeted liposomal doxorubicin clearly surpasses the concentration obtained with free doxorubicin HPLC analysis of drug accumulated in cells exposed to folate-targeted liposomes shows that the accumulated drug is intact drug (data not shown), indicating that this route of delivery does not lead to drug degradation. Also, no significant amounts of metabolites were detected after 1 to 4 hours exposure of tumor cells to either free doxorubicin or folate-targeted liposomal doxorubicin.

### 3. Cytotoxicity

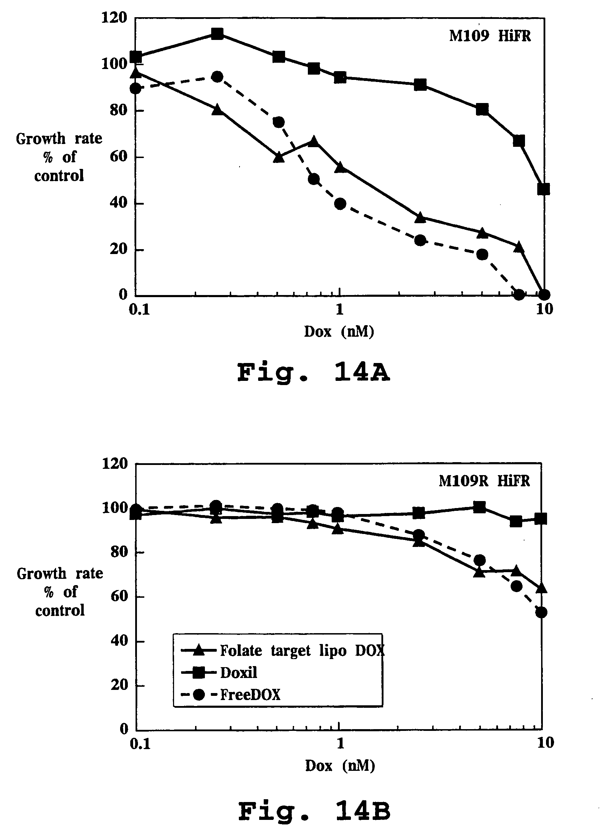

The cytotoxicity doxorubucin delivered to M109-HiFR cells in free form and in folate-targeted liposome-entrapped form was compared as described in Example 8. Briefly. M109-HiFr cells were exposed to free or liposomal doxorubicin for 1 hour, then washed and further incubated for 5 days (120 hours) in fresh medium. As seen in Fig. 14A, the growth inhibition curve of doxorubicin in folate-targeted liposomes (closed triangles) is similar to that for doxorubicin administered in free form (closed circles), and considerably higher to that of doxorubicin when administered in the form of conventional, non-targeted liposomes (closed squares).

A similar cytotoxicity assay was done using the multi-drug resistant subline, M109R-HiFR and the results are shown in Fig. 14B. A clear enhancement of cytotoxicity was obtained when the drug was administered to the cells via folate-targered liposomes (closed triangles) when compared to drug administered from conventional, non-targeted liposomes (closed squares).

### II. In vivo Characterization of the Composition

To examine the biological activity of drug delivered by the folate-pathway from folate-targeted liposomes in another model, M109R-HiFR cells *in vitro* were exposed to a test drug. These cells were inoculated into mice footpads. In this way, the growth of cells was tracked along a much longer time span than in *in* vitro experiments, and the influence of *in vivo* micro-environmental factors is brought into play. However, unlike therapeutic experiments, this type of *in vivo* adoptive assay is unaffected by pharmacokinetic and biodistribution factors which complicated the interpretation of results.

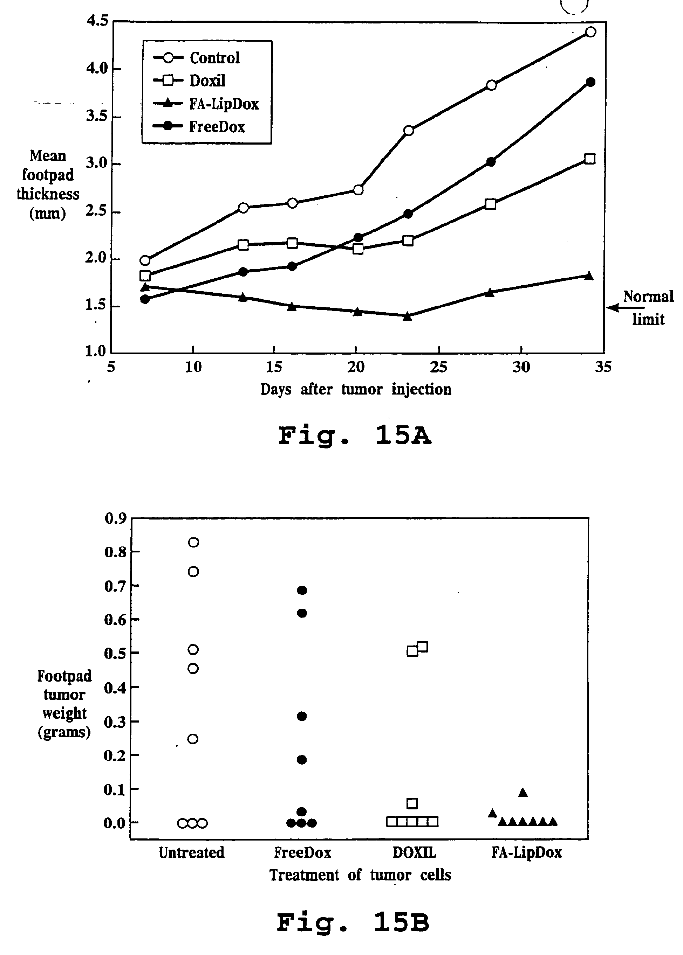

Accordingly, tumor cells *in vitro* were incubated with free doxorubicin or with liposome-entrapped, non-folate-targeted doxorubicin (DOXIL®) or with folate-targeted, liposome-entrapped doxorubicin in accord with the invention. As described in Example 9, the tumor cells were incubated in the presence of the selected formulation for 1 hour and then 1 x 10⁶ cells were injected into the footpad of a mouse, each treatment formulation being injected into 8 mice. The footpad thickness of each mouse of each of the treatment groups was measured, and the results are shown in Fig. 15A.

As seen in the figure, the control mice (open circles), that is the mice injected with tumor cells not treated with doxorubicin, had a continual increase in footpad thickness after injection of the tumor cells. Mice receiving cells treated with free doxorubicin (closed circles) and mice receiving tumor cells treated with liposome-entrapped doxorubicin (open squares) also experienced an increase in footpad thickness. Only the mice injected with cells treated with the folate-targeted liposomes (closed triangles) had no increase in footpad thickness.

At the end of the study on day 34 after injection of the treated tumor cells, the weight of each tumor-bearing mouse footpad was determined and subtracted from the average weight of a mouse footpad to determine the tumor weight in each mouse footpad. Fig. 15B shows the footpad tumor weight in grams for each of the treatment regimens. As seen, the tumors in the mice receiving the cells treated with the folate-targeted doxorubicin-entrapped liposomes (closed triangles) had the smallest average tumor weight.

Table 5 summarizes the tumor incidence and the media tumor weight for each of the treatment groups.

**Table 5**

| Treatment | Final Tumor Incidence^{c} | Median Tumor weight (range) |
|---|---|---|
| | (%) | (mg) |
| Untreated | 13/20 (65 %) | 381 (48-825) |
| Free DOX^{a} | 8/19 (42 %) | 239 (32-683) |
| DOXIL®^{,b} | 10/19 (53 %) | 397 (13-512) |
| folate-lipo-DOX | 2/20 (10 %) | 57 (27-87) |

| | | |
|---|---|---|
| ^{a}DOX=doxorubicin ^{b}results of two experiments ^{c}Fisher's exact test: folate-targeted-DOX vs. Untreated, p=0.0008; folate-targeted-DOX vs. DOXIL, p=0.0057; folate-targeted-DOX vs. Free DOX, p=0.0310. All other comparisons, not significant. | | |

The results in Table 6 point to a statistically significant decrease of the number of tumor occurrences in mice injected with tumor cells exposed to folate-targeted liposomal doxorubicin, as compared to free doxorubicin, DOXIL, and control, after 5 weeks follow-up. Tumor weights were also smaller for the folate-targeted liposomal group.

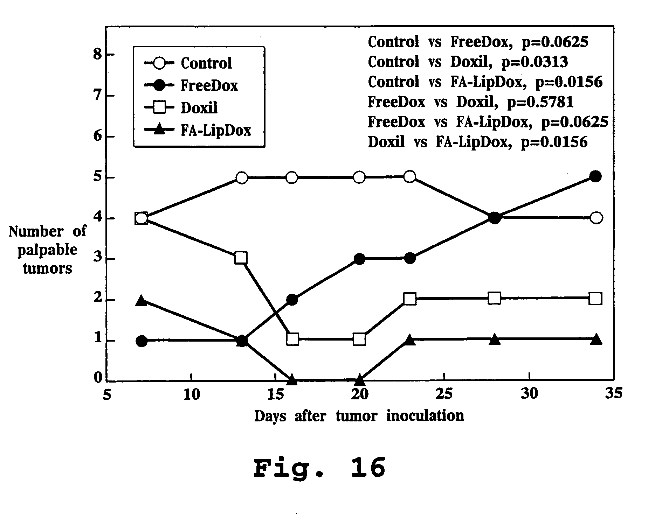

In a similar study, tumor cells treated with each of the formulation were injected subcutaneously into mice and the number of palpable tumors as a function of time after injection was determined. The results are shown in Fig. 16, and the control mice (open circles) had the highest number of tumors. The mice receiving the free doxorubicin treated cells (closed circles), after a slow initial tumor growth phase, also had a significant number of tumors after about day 20. The mice receiving the liposome formulations (open squares for non-targeted liposomes and closed triangles for folate-targeted liposomes) had the fewest tumors, with the folate-targeted liposome doxorubicin resulting in the fewest number of tumors.

### III. Examples

The following examples illustrate methods of preparing and characterizing the liposome composition of the invention and in no way are intended to be limiting.

### Example 1

### Preparation and Characterization of Folic Acid-PEG-DSPE Conjugates

### A. Synthesis of Conjugate

Folic acid (Fluka, 100 mg, 0.244 mmol) was dissolved in DMSO (4mL). Amino-PEG₂₀₀₀-DSPE (prepared as set forth in Zalipsky, S. et al., FEBS Lett. 353:71-74 (1994)) (400 mg, 0.14 mmol) and pyridine (2 mL) were added to the folic acid-DMSO solution followed by dicyclohexylcarbodiimide (130 mg, 0.63 mmol). The reaction was continued at room temperature for 4 hours. TLC on silica gel GF (chloroform/methanol/water 75:36:6) showed a new spot (R₁ = 0. 57) due to the formation of the product. Disappearance of amino-PEG-DSPE (R₁ = 0.76) from the reaction mixture was confirmed by ninhydrin spray. Pyridine was removed by rotary evaporation. Water (50 mL) was added to the reaction mixture. The solution was centrifuged to remove trace insolubles. The supernatant was dialyzed in Spectra/Por CE (Spectrum, Houston, TX) tubing (MWCO 300,000) against saline (50 mM, 2 x 2000 mL and water (3 x 2000 mL). The resulting solution, containing only the product (single spot by TLC) was lyophilized and the residue dried *in vacuo* over P₂O₅. Yield: 400 mg, 90%. The synthesis is illustrated in Fig. 1.

The same protocol was used to prepare folic acid-PEG-DSPE from H₂N-PEG₃₃₅₀-DSPE. In a similar procedure folic acid was attached to mPEG₂₀₀₀-NH₂ (Zalipsky, S., et al., Eur. Polym. J., 19:1177-1183 (1983)). The product, mPEG-folic acid, was purified on silica gel (70-200 mesh, 60 Å) column using stepwise gradient of methanol (10 - 80%) in chloroform and then chloroform / methanol / water (65: 30 : 5) for the elution of the pure product.

### B. Characterization of the Conjugate by UV Analysis

Folate content value was determined by quantitative UV spectrophotometry of the conjugates in methanol (0.05 mg/mL) using folic acid extinction coefficient ε = 27,500 M⁻¹ .cm⁻¹ at λₘₐₓ = 285 nm. The following folic acid content values were calculated: 0.29 mmol / g (94 % of theoretical value, 0.31 mmol/g) for folic acid-PEG₂₀₀₀-DSPE; 0.21 mmol/g (97 % of theoretical value, 0.22 mmol/g) for folic acid-PEG₃₃₅₀-DSPE, and 0.40 mmol/g (98 % of the theoretical value, 0.41 mmol/g) for mPEG₂₀₀₀-folic acid.

### C. Characterization of the Conjugate by ¹H-NMR (360 MHz, DMSO-D6 / CF₃CO₂D ~10/1 v/v)

For folic acid-PEG-DSPE: δ 0.84 (t, CH₃, 6H); 1.22 (s, CH₂, 56H); 1.49 (m, CH₂CH₂CO, 4H); 2.1-2.3 (overlapping 2 x τ, CH₂CH₂CO & m CH₂ of Glu, 8H); 3.2 (m, CH₂CH₂N, 4H); 3.50 (s, PEG, ~180H and -300H for derivatives of PEG2000, and -3350 respectively); 4.02 (t, *CH*₂OCONH, 2H); 4.1 (dd, trans-PO₄*CH*₂CH, 1H); 4.3 (dd, cis-PO₄*CH*₂CH 1H); 4.37 (m, α-CH, 1H); 4.60 (d, 9-CH₂-N, 2H); 5.15 (M, PO₄CH₂*CH* 1H); 6.65 (d, 3',5'-H,2H); 7.65 (d, 2',6'-H); 8.77 (s, C7-H, 1H) ppm. For mPEG-folic acid: δ 1.85-2.1 (m, β-CH₂ of Glu, 2H); 2.3 (m, γ- CH₂ of Glu, 2H);); 3.11 (m, CH₂CH₂N, 4H); 3.50 (s, PEG, -180H); 4.3 & 4.37 (minor & major α-CH₂ of Glu, 1H); 4.60 (9-CH₂-N, 2H);); 6.65 (d, 3', 5'-H, 2H); 7.66 (d, 2',6'-H,2H); 8.77 (s, C7-H, 1H) ppm.

### D. Characterization of the Conjugate by Mass Spectra (MALDI-TOFMS)

The spectra were obtained by Charles Evans & Associates (Redwood City, CA) with PHI-EVANS MALDI triple electrostatic analyzer time-of-flight mass spectrometer (desorption laser: 337 nm, 600 psec pulse width), utilizing gentrinsic acid as a matrix material. The spectra exhibited a bell-shaped distributions of 44 DA-spaced lines centered at 3284 for folic acid-PEG2000-DSPE (calculated molecular weight 3200 Da); 4501 for folic acid-PEG3350-DSPE (calculated molecular weight 4540 Da); and at 2400 for mPEG-folic acid (calculated molecular weight 2423 Da).

### E. HPLC monitoring of carboxypeptidase G-mediated Cleavage

A HPLC system, Shimadzu 10 A, equipped with Phenomenex Prodigy C8 (4.6.50 mm) column was used at 1 mL/min, while monitoring λ = 285 nm. For analysis of folic acid-PEG-DSPE the system was used in isocratic mode, methanol / 10 mM sodium phosphate; pH 7.0 (92:8, v/v). The conjugate eluted as a single peak with a retention time of 5.7 min. Analysis of mPEG-folic acid was performed by a gradient mode, using 10 mM sodium phosphate, pH 7.0 with methanol (0-35 % in 25 min). The conjugate eluted as a single peak with retention time of 19.6 min. In both cases it was possible to follow the enzymatic cleavage of pteroate from the folic acid-moiety of the conjugates by the decrease in the total conjugate peak area. A solution of folic acid-PEG-DSPE (0.1 mg/ml) was prepared in 150 mM Tris buffer, pH 7.3. An aliquot (10 µL) of this solution was injected into HPLC to obtain the time zero peak integration. The enzyme carboxypeptidase G (CPG, Sigma, one unit) was added to the folic acid-PEG-DSPE solution. The resulting solution was incubated at 30 °C water bath and aliquots (10 µL) were injected into the HPLC at different time intervals. The rate of enzymatic hydrolysis was initially rapid the slowing after 4 hours of incubation time. Additional one unit aliquots of CPG were added to the reaction mixture at 4 hours and 20 hours. Data was collected for up to 27 hours. Despite the prolonged incubation times and multiple additions of the enzyme the disappearance of the conjugate peak did not exceeded 20% of the initial integration value, indicating that 80% of the folic acid-PEG-DSPE was γ-carboxyl linked. The experiment performed with mPEG-folic acid as a substrate showed that this conjugate contained ~90% of folic acid residues γ-carboxyl linked.

### Example 2

### Cell Culture and Binding Studies

### A. Cell Culture

Cells were cultured in normal or folic acid-free RPMI medium, with 10% fetal bovine serum, glutamine 2mM, penicillin 50 u/mL, and streptomycin 50 µg/mL. The concentration of folic acid in the serum-containing folic acid-free medium is only 3 nM, as opposed to 2.26 µM (1 mg/L) under normal culture conditions. Cells were routinely passed by treatment with trypsin (0.05%) - EDTA (0.02%) solution in Industries (Beyt Haemek, Israel), and fetal bovine serum was from GIBCO (Grand Island, NY).

(i) Cell lines: M109, a murine lung carcinoma line of BALB/c mice (Marks, T.A. et al., Cancer Treat. Rep. , 61:1459-1470 (1997)), and a subline of these cells, M109R displaying multidrug-resistance, (approximately 100 fold increased resistance to doxorubicin) were used in most of the studies. Both cell lines express *in vitro* low amounts of folic acid receptors and are therefore referred to as M 109-LoFR and M109R-LoFR. By culturing these cells in folic acid-free medium for several passages, two sublines expressing a high amount of folic acid receptors were obtained. These sublines were adapted to grow under low folic acid conditions with generation doubling times similar to the lines of origin. These sublines are referred to as M109-HiFR and M109R-HiFR, to emphasize the over-expression of folic acid receptors.

KB cells, a human nasopharyngeal epidermal carcinoma (Saikawa, Y., Biochemistry, 34:9951-9961 (1995)), were also grown in low folic acid medium to obtain cells over-expressing folic acid receptors, KB-HiFR cells. Other cell lines used in this study were A375, a human melanoma line, and normal human fibroblasts which were kindly provided by the Genetics Department of Hadassah Hebrew University Hospital.

### B. Cell Binding of Free Folate, Folic Acid-PEG Conjugates and Liposomes

Binding was assayed through measurement of cell-associated liposomal ³H-Chol or ³H-folic acid. 48 hours prior to an assay, 5x10⁵ cells were seeded in a 35 mm dish, to obtain about 10⁶ cells/plate. Plates preincubated with medium and serum for 2 days, without cells, were used as controls. For the assays, plates were washed twice with folic acid-free RPMI medium, and incubated at 37 °C with 1 mL of folic acid-free RPMI medium containing 0.1 µM radiolabeled folic acid or liposomes in amounts of 30-300 nmoles phospholipid. After incubation, the plates were rinsed 3 times with 2 mL ice-cold PBS, and the radiolabels were extracted with 1 mL of 0.5 N NaOH overnight, followed by neutralization with 1 mL 0.5 N HCL. To analyze radioactivity associated with cells, cells were released from plates by trypsin-EDTA treatment, washed 3 times with PBS, and then extracted following the same procedure. Radioactivity was determined by liquid scintillation counting. Based on the specific ratio of cpm/phospholipid for each liposome formulation, the results were translated into picomoles phospholipid per million cells.

For acid wash treatment following binding, each dish was washed twice with acidified saline (pH=3), followed by wash with PBS, and then extracted as described above.

For treatment of cells with phosphatidylinositol-phospholipase C (PI-PLC), M109 HiFR cells were rinsed twice with folate-free RPMI medium were exposed to 0.1 u/mL phosphoinositol phospholipase-C (PI-PLC) (Boehringer, Mannheim) in folate-free RPMI medium for 60 minutes at 37°C. Subsequently, the cells were rinsed again twice with folate-free RPMI medium and exposed to folate-targeted liposomes for 1 hour at 4°C. Microscopic examination was done with fixed cell samples.

In studies performed with cells in suspension, cells from monolayers were released by trypsin - EDTA treatment, followed by three washes (7 min, 500g centrifugation) in folic acid-free RPMI medium. The suspended cells (1-10x10⁶ cells/mL) were incubated with radiolabeled free folic acid or liposomes (concentration as indicated for each study) for 30 min in 5-mL plastic tubes. Unbound material was removed by four washes with PBS.

### Example 3

### Liposome Preparation

Liposomes composed of hydrogenated soybean phosphatidylcholine (HSPC) (Avanti Polar Lipids, Birmingham, AL), cholesterol (Chol) (Sigma, St. Louis, MO) and methoxyPEG₂₀₀₀-DSPE (mPEG-DSPE) were prepared as described previously (Zalipsky, S., et al, Bioconjugate Chem. , 4:296-299 (1993)). The liposome compositions are set forth in Table 2, above and, as discussed, because all of the formulations contained HSPC, Chol, and DSPE, thus, they are referred to herein according to folic acid-PEG/mPEG content. Fig. 3 schematically illustrates the formulations.

All liposome preparations were spiked with a trace amount of ³H-Cholhexadecyl ether (NEN, Boston, MA). Liposomes were made by hydration at 55-60 °C of either a thin dry lipid film obtained by rotary evaporation of a chloroform:methanol (1:1) lipid solution or a freeze-dried lipid "cake" obtained by lyophilization of tert-butanol lipid solution. The buffer used was 5 % dextrose/ 15 mM Hepes, pH 7.4 at a concentration of 50-100 µmoles phospholipid/mL. Hydration was followed by high-pressure extrusion through double-stacked polycarbonate membranes with pore sizes from 1.0 to 0.05 µm using a stainless steel device from Lipex Biomembranes (Vancouver, BC). Liposomes were sterilized by filtration through 0.22 µm cellulose membranes. Liposome characterization included: phospholipid concentration based on phosphorus content, folic acid concentration based on the UV absorbance of folic acid at 285 nm after liposome disruption in sodium dodecyl sulfate solution (10%), vesicle size as determined by dynamic laser scattering, and, in some preparations, free fatty acid content to check for phospholipid hydrolysis. All liposome preparations had a mean vesicle size between 70-90 nm with a standard deviation < 25 % and a unimodal size distribution. Phospholipid hydrolysis was not detected in the preparations tested here.

### Example 4

### Binding and Internalization of Folate-targeted Liposomes to M109 HiFR Cells

### A. Liposome Preparation

Liposomes were prepared according to the procedure of Example 3 to include DPPE-rhodamine (Avanti Polar Lipids, Birmingham, AL) as follows:

| **Formulation** | **Liposome Component Molar Percent** | | | | |
|---|---|---|---|---|---|
| | **HSPC** | **Chol** | **DSPE-PEG₂₀₀₀-Folate** | **mPEG-DSPE₂₀₀₀** | **DPPE-rhodamine** |
| Rh¹-folic acid-PEG₂₀₀₀ | 98.9 | 70 | 1.0 | 0 | 0.1 |
| Rh-folic acid-PEG₂₀₀₀/mPEG | 91.9 | 70 | 1.0 | 7 | 0.1 |

| | | | | | |
|---|---|---|---|---|---|
| ¹Rh=rhodamine | | | | | |

### B. Confocal Microscopy

M109 HiFR cells were plated, 24 hours prior to each study, on 24 mm cover slips inserted into 35mm culture dishes. Exposure times of the cells to the liposome composition or to free doxorubicin are indicated for each study. Cells were fixed with the buffered, PBS solution containing 4 % formalin / 1.5 % methanol (Bio-Labs, Israel) at 4°C for 15 minutes, then washed 4 times with PBS (Gibco, Grand Island, NY). Next, the cover slips were put on slides coated with buffered mounting medium consisting of 90% glycerol/10% PBS/0.1 % NaN₃ and 3 % DABCO (Sigma) as anti-fading agent.

Microscopic visualization of live (non fixed) cells was done in PBS containing 2mM MgSO₄/1mM HEPES (Sigma), pH 7.5.

Examination of the cells was done with inverted Zeiss confocal laser scanning microscope (LSM410) (Carl Zeiss, Jena, Germany). Maximum excitation was done by 543nm line of the internal He-neon laser; fluorescence emission was observed above 570nm with long-pass barrier filter LP-570) for rhodamine. For doxorubicin, maximum excitation was done by 488nm line of internal Argon laser: fluorescence emission was observed above 515nm with long pass barrier filter LP-515. A water immersion objective C-Apochromat 63x1.2 W corr. (Zeiss) was used. Images were converted to TIF file format, and the contrast level and brightness of the images were adjusted by using the Zeiss LSM410 program. The images were printed from QMS magicolor 2 printer at 1200 dpi.

### Example 5

### Preparation of Doxorubicin Liposomes and In vitro Binding

### A. Liposome Preparation

Preparation of liposomes was carried out as described by Gabizon (J. Drug Targeting, 3:391-398, (1996), and were composed of hydrogenated soybean phosphatidylcholine (HSPC, Avanti Polar Lipids, Birmingham-LA, USA), cholesterol (Sigma), DSPE-PEG-Folate. The doxorubicin to phospholipid ratio was between 110-150 µg/µmol. Doxorubicin-loaded liposomes lacking the folate targeting ligand, but having a surface coating of PEG, were as described in Cabanes, A., et al., Clinical Cancer Res. 4:499-505, (1998), and as sold under the tradename DOXIL (Sequus Pharmaceuticals, Inc.).

### B. In vitro Binding

M109R-HiFr cells were incubated with free doxorubicin or with doxorubicin entrapped in folate-targeted liposomes at a doxorubicin concentration of 4x10⁻⁵ M. The doxorubicin molecule was tracked using fluorescence.

### Example 6

### Verapamil Blockade of Efflux Pump and Delivery of Doxorubicin

Monolayers of M109R HiFR cells in 35mm culture dishes were exposed to 0.5x10 ⁵M doxorubicin as free drug or in folate-targeted liposomes for 1 hour at 37 °C, in the presence or absence of 10 µmol verapamil (Teva, Israel) followed by PBS washing (7 min, 500g centrifugation). Then, the washed cells
were rinsed and further incubated with verapamil for 2 hours. Cells were released from monolayer with 0.05% trypsin/0.02 % EDTA (Gibco, Grand Island, NY), and were split into two fractions, one fraction for cellular doxorubicin determination using fluorescence and the other fraction for flow cytometry assay. Cellular doxorubicin determination was determined by extracting the doxorubicin with 0.075N HCI/ 90 % isopropyl alcohol at 4°C overnight, centrifuging and assaying the supernatant collection for doxorubicin by fluorescence using a fluorimeter (Kontron, Lumitron, Israel) at Ex 470nm; Em590nm.

The flow cytometry assay was performed as follows. Suspended M 109R-HiFR cells as described above, were analyzed by flow cytometry using a FACS-Star Plus (Becton-Dickinson, Immunofluorometry System, Mountain View, CA) flow cytometer. Cells were passed at a rate of approximately 1000 cells/see through a 70 µm nozzle, using saline as the sheath fluid. A 488nm argon laser beam at 250mW served as the light source for excitation. Red (doxorubicin derived) fluorescence was measured using a 575nm DF 26 band-pass filter.

The results are shown in Figs. 11A-11B and in Table 4.

### Example 7

### Cellular and Nuclear Doxorubicin Quantitation

M109-HiFR and M109R-HiFR cells were exposed to free doxorubicin or to doxorubicin entrapped in folate-targeted liposomes for 1 hour and 4 hours. Quantitation of drug accumulating in the cells was done fluorometrically on trypsinized cells as described above in Example 6. Doxorubicin exposed M109R-HiFR cells typsinized and PBS washed were suspended for 10min at 4°C, in the following solution: 100mM NaCl/1mM EDTA/1%Triton X-100 (Sigma)/10mM Tris (Sigma), pH 7.4, then centrifuged (15min, 800g). Cell nuclei precipitate was separated from cell cytosol and doxorubicin was extracted from both fractions as described in Example 6. The results are shown in Fig 13.

### Example 8

### Cytotoxicity Studies

M 109 HiFR and M109R HiFR cells in folate-free RPMI medium seeded in 96 well plates (6 replicates) at a density of 10³ cells/well, were exposed for 1 hour to doxorubicin in free form, in non-targeted liposome-entrapped form and in folate-targeted liposome-entrapped form. After exposure the cells were rinsed twice and incubated further for 120 hours in the above medium. Cell growth assay was done using 2.5 % glutaraldehyde (Merck) as fixative, followed by methylene blue (Merck) staining, and absorbance measurements at 620nm on an automated plate reader. The results are shown in Figs. 14A-14B.

### Example 9

### In vivo Adoptive Tumor Growth Assay

Female 10-week-old BALB/c mice were maintained in a specific pathogen-free facility. M109R-HiFR cells in *in vitro* suspension (10⁷ cells/ml) were exposed to 10⁻⁵M doxorubicin either as free drug, as liposome-entrapped (Doxil®), or as folate-targeted liposome-entrapped for 2 hours, washed with PBS, and then resuspended at a concentration of 2x10⁷ cells. Healthy, syngeneic BALB/c mice were injected into the right hind footpad with 50µl (10⁶ cells). The footpad thickness was measured with calipers once or twice a week for 5 weeks. After 35 days, mice were sacrificed, the final number of tumors recorded, and the control and tumor-inoculated footpads were sectioned at the ankle level and weighed. Tumor weight was estimated as the difference between the weight of the normal and tumor-bearing footpad. The statistical significance of differences in the final incidence of tumors per group was analyzed by contingency tables and the Fisher's exact test. The results are shown in Figs. 15A-15B and Fig. 16 and in Table 5.

Although the invention has been described with respect to particular embodiments, it will be apparent to those skilled in the art that various changes and modifications can be made without departing from the invention.

## Claims

1. The use of a composition consisting of:
a liposome carrier composed of hydrogenated soybean phosphatidylcholine (HSPC), cholesterol, and distearoyl phosphatidyl ethanolamine-polyethylene glycol-folate (DSPE-PEG-folate) and
doxorubicin entrapped in the liposome carrier, wherein the doxorubicin to phospholipid ratio is between 110-150 µg/µmol
in the manufacture of a medicament for the treatment of multi-drug resistant cancer.

## Patentansprüche

1. Verwendung einer Verbindung, bestehend aus:
einem Liposomenträger zusammengesetzt aus hydriertem Sojabohnen-Phosphatidylcholin (HSPC), Cholesterin und Distearoylphosphatidylethanolaminpolyethylenglycol-folat (DSPE-PEG-Folat) und
Doxorubicin, eingeschlossen in dem Liposomenträger, wobei das Verhältnis Doxorubicin zu Phospholipid 110 - 150 µg/pmol beträgt,
zur Herstellung eines Medikaments zur Behandlung von mehrfachresistentem Krebs.

## Revendications

1. Utilisation d'une composition constituée :
d'un support liposomique composé de phosphatidylcholine hydrogénée de soja (HSPC), de cholestérol, et de distéaroyl-phosphatidyl-éthanolamine-polyéthylène glycol-folate (DSPE-PEG-folate) et
de doxorubicine renfermée dans le support liposomique, où le rapport de la doxorubicine sur le phospholipide est entre 110 et 150 µg/µmol
dans la fabrication d'un médicament destiné au traitement d'un cancer multirésistant aux médicaments.
